# EUROPEAN PATENT APPLICATION

(11) **EP 3 884 846 A1**
(43) Date of publication of application: **29.09.2021**
(21) Application number: 19905742.3
(22) Date of filing: 13.09.2019
(51) Int. Cl.: A61B 3/113, A61B 10/00, G09G 5/00

(54) **DISPLAY DEVICE, DISPLAY METHOD, AND PROGRAM**

(30) Priority: 28.12.2018 JP 2018248420
(71) Applicant: JVCKenwood Corporation, Yokohama-shi, Kanagawa 2210022 (JP)
(72) Inventor: HAKOSHIMA, Shuji, Yokohama-shi, Kanagawa 221-0022 (JP); SHUDO, Katsuyuki, Yokohama-shi, Kanagawa 221-0022 (JP)
(74) Representative: Schmidbauer, Andreas Konrad
(86) International application number: PCT/JP2019/036112
(87) International publication number: WO 2020/137028

(57) **Abstract**

A display device (101) includes a storage unit (222) configured to store positional data of a gaze point of a subject with respect to a video, an arithmetic unit (220) configured to calculate gaze information that visually indicates the gaze point from the positional data, and a display control unit (202) configured to cause a display screen of a display unit (101S) to display the gaze information in a superimposed manner on the video in accordance with a replay elapsed time of the video.

## Description

### Field

The present disclosure relates to a display device, a display method, and a program

### Background

Conventionally, as a technology for examining gaze tendencies of lines of sight of a large number of subjects at a time, a technology for examining a distribution of the lines of sight from pieces of coordinate data of measured gaze points has been known.

For example, Patent Literature 1 describes a technology capable of calculating a time during which a gaze point of a subject is present in a specific area including a feature point in image data.

### Citation List

### Patent Literature

Patent Literature 1: Japanese Laid-open Patent Publication No. 2017-158866

### Summary

However, in Patent Literature 1, it is difficult to inspect a gaze tendency of the subject with respect to a specific content of the image data. Therefore, a technology capable of inspecting the gaze tendency of the subject has been demanded.

An object of the present disclosure is to provide a display device, a display method, and a program capable of inspecting gaze tendencies by displaying a distribution of lines of sight of subjects with respect to a video in addition to the video.

A display device according to the present disclosure includes a storage unit configured to store positional data of a gaze point of a subject with respect to a video; an arithmetic unit configured to calculate gaze information that visually indicates the gaze point from the positional data; and a display control unit configured to cause a display screen of a display unit to display the gaze information in a superimposed manner on the video in accordance with a replay elapsed time of the video.

A display method according to the present disclosure includes a calculation step of calculating gaze information that visually indicates a gaze point from positional data of the gaze point of a subject with respect to a video; and a display control step of causing a display screen of a display unit to display the gaze information in a superimposed manner on the video in accordance with a replay elapsed time of the video.

A program according to the present disclosure causes a computer to execute a calculation step of calculating gaze information that visually indicates a gaze point from positional data of the gaze point of a subject with respect to a video; and a display control step of causing a display screen of a display unit to display the gaze information in a superimposed manner on the video in accordance with a replay elapsed time of the video.

According to the present disclosure, it is possible to provide a display device, a display method, and a program capable of inspecting a gaze tendency of a subject with respect to image data.

### Brief Description of Drawings

FIG. 1 is a perspective view schematically illustrating an example of a line-of-sight detection apparatus according to the present embodiment.
FIG. 2 is a diagram illustrating an example of a hardware configuration of the line-of-sight detection apparatus according to the present embodiment.
FIG. 3 is a functional block diagram illustrating an example of the line-of-sight detection apparatus according to the present embodiment.
FIG. 4 is a schematic diagram for explaining a method of calculating positional data of a corneal curvature center according to the present embodiment.
FIG. 5 is a schematic diagram for explaining the method of calculating the positional data of the corneal curvature center according to the present embodiment.
FIG. 6 is a schematic diagram for explaining an example of a calibration process according to the present embodiment.
FIG. 7 is a schematic diagram for explaining an example of a gaze point detection process according to the present embodiment.
FIG. 8 is a schematic diagram for explaining an example of conventional gaze point display.
FIG. 9 is a schematic diagram for explaining an example of a conventional screen for dynamically displaying gaze points.
FIG. 10 is a schematic diagram illustrating an example of a data set related to gaze data of the subject according to the present embodiment.
FIG. 11A is a schematic diagram for explaining an example of a method of displaying gaze points on a display screen according to the present embodiment.
FIG. 11B is a schematic diagram for explaining an example of the method of displaying the gaze points on the display screen according to the present embodiment.
FIG. 11C is a schematic diagram for explaining an example of the method of displaying the gaze points on the display screen according to the present embodiment.
FIG. 11D is a schematic diagram for explaining an example of the method of displaying the gaze points on the display screen according to the present embodiment.
FIG. 11E is a schematic diagram for explaining an example of the method of displaying the gaze points on the display screen according to the present embodiment.
FIG. 12 is a schematic diagram for explaining an example of a method of displaying gaze points on the display screen according to the present embodiment.
FIG. 13 is a schematic diagram for explaining an example of the method of displaying gaze points on the display screen according to the present embodiment.
FIG. 14 is a flowchart illustrating an example of the flow of a process of displaying gaze points on a video.
FIG. 15 is a schematic diagram for explaining an example of a method of selecting a subject to be displayed on a video.
FIG. 16 is a schematic diagram for explaining a method of selecting a type of gaze information to be displayed on a video.
FIG. 17 is a flowchart illustrating an example of the flow of a process of detecting gaze points.
FIG. 18 is a flowchart illustrating an example of the flow of a process of performing multiple display of gaze points on a video.
FIG. 19 is a flowchart illustrating an example of the flow of a process of displaying a heat map of gaze points on a video.
FIG. 20 is a flowchart illustrating an example of the flow of a process of displaying gaze rates for each of grids on a video.

### Description of Embodiments

Embodiments according to the present disclosure will be described below with reference to the drawings, but the present disclosure is not limited to the embodiments below. Structural elements of the embodiments below may be combined appropriately. In addition, some structural elements are not used in some cases.

In the following description, a three-dimensional global coordinate system is set and positional relationships among components will be described. A direction parallel to a first axis of a predetermined plane is referred to as an X-axis direction, a direction parallel to a second axis that is perpendicular to the first axis in the predetermined plane is referred to as a Y-axis direction, and a direction parallel to a third axis that is perpendicular to the first axis and the second axis is referred to as a Z-axis direction. In the present embodiment, the predetermined plane is an XY plane.

### Line-of-sight detection apparatus

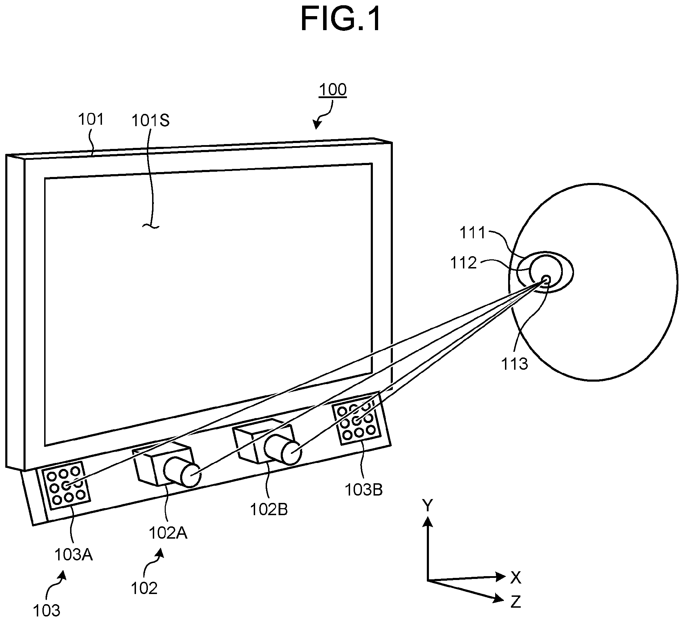

FIG. 1 is a perspective view schematically illustrating an example of a line-of-sight detection apparatus 100 according to a first embodiment. The line-of-sight detection apparatus 100 is used as an evaluation apparatus that evaluates whether a probability of a developmental disability is high or low. As illustrated in FIG. 1, the line-of-sight detection apparatus 100 includes a display device 101, a stereo camera device 102, and a lighting device 103.

The display device 101 includes a flat panel display, such as a liquid crystal display (LCD) or an organic electroluminescence display (OLED). In the present embodiment, the display device 101 includes a display unit 101S. The display unit 101S displays an image. In the present embodiment, the display unit 101S displays an index for evaluating visual performance of a subject, for example. The display unit 101S is substantially parallel to the XY plane. The X-axis direction corresponds to a horizontal direction of the display unit 101S, the Y-axis direction corresponds to a vertical direction of the display unit 101S, and the Z-axis direction is a depth direction perpendicular to the display unit 101S.

The stereo camera device 102 includes a first camera 102A and a second camera 102B. The stereo camera device 102 is arranged below the display unit 101S of the display device 101. The first camera 102A and the second camera 102B are arranged in the X-axis direction. The first camera 102A is arranged in the negative X direction relative to the second camera 102B. Each of the first camera 102A and the second camera 102B includes an infrared camera that includes, for example, an optical system capable of transmitting near-infrared light with a wavelength of 850 nanometers (nm) and an imaging element capable of receiving the near-infrared light.

The lighting device 103 includes a first light source 103A and a second light source 103B. The lighting device 103 is arranged below the display unit 101S of the display device 101. The first light source 103A and the second light source 103B are arranged in the X-axis direction. The first light source 103A is arranged in the negative X direction relative to the first camera 102A. The second light source 103B is arranged in the positive X direction relative to the second camera 102B. Each of the first light source 103A and the second light source 103B includes a light emitting diode (LED) light source, and is able to emit near-infrared light with a wavelength of 850 nm, for example. Meanwhile, the first light source 103A and the second light source 103B may be arranged between the first camera 102A and the second camera 102B.

The lighting device 103 emits near-infrared light as detection light, and illuminates an eyeball 111 of the subject. The stereo camera device 102 causes the second camera 102B to capture an image of a part of the eyeball 111 (hereinafter, the part is also referred to as the "eyeball") when the eyeball 111 is irradiated with the detection light emitted from the first light source 103A, and causes the first camera 102A to capture an image of the eyeball 111 when the eyeball 111 is irradiated with the detection light emitted from the second light source 103B.

At least one of the first camera 102A and the second camera 102B outputs a frame synchronous signal. The first light source 103A and the second light source 103B emit the detection light on the basis of the frame synchronous signal. The first camera 102A captures image data of the eyeball 111 when the eyeball 111 is irradiated with the detection light emitted from the second light source 103B. The second camera 102B captures image data of the eyeball 111 when the eyeball 111 is irradiated with the detection light emitted from the first light source 103A.

If the eyeball 111 is irradiated with the detection light, a part of the detection light is reflected by a pupil 112, and the light from the pupil 112 enters the stereo camera device 102. Further, if the eyeball 111 is irradiated with the detection light, a corneal reflection image 113 that is a virtual image of a cornea is formed on the eyeball 111, and light from the corneal reflection image 113 enters the stereo camera device 102.

By appropriately setting relative positions between the first camera 102A/the second camera 102B and the first light source 103A/the second light source 103B, intensity of light that enters the stereo camera device 102 from the pupil 112 decreases, and intensity of light that enters the stereo camera device 102 from the corneal reflection image 113 increases. In other words, an image of the pupil 112 captured by the stereo camera device 102 has low luminance, and an image of the corneal reflection image 113 has high luminance. The stereo camera device 102 is able to detect a position of the pupil 112 and a position of the corneal reflection image 113 on the basis of luminance of a captured image.

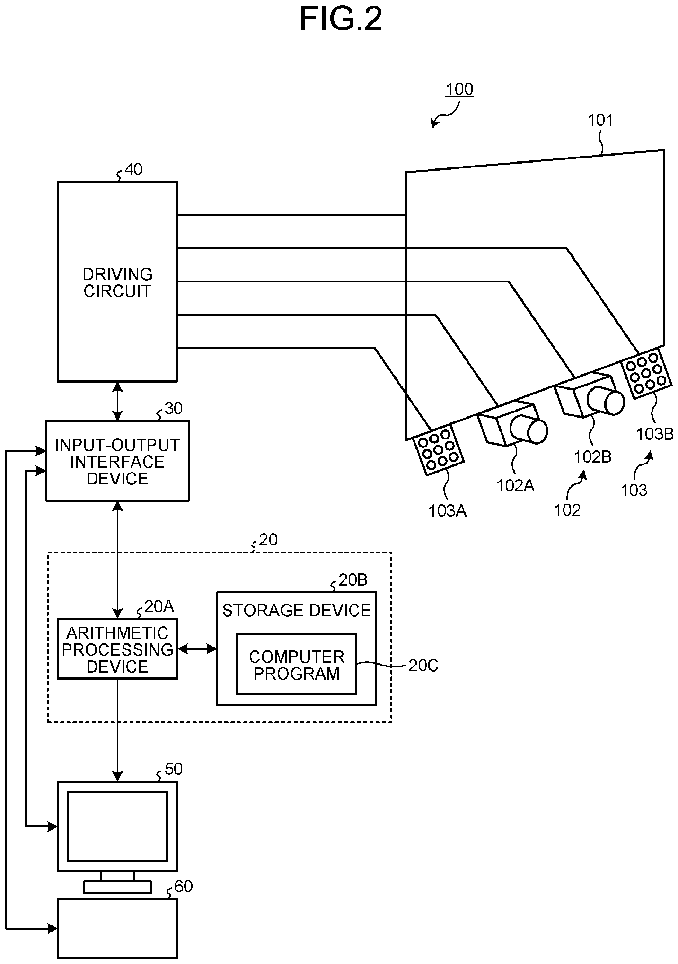

FIG. 2 is a diagram illustrating an example of a hardware configuration of the line-of-sight detection apparatus 100 according to the present embodiment. As illustrated in FIG. 2, the line-of-sight detection apparatus 100 includes the display device 101, the stereo camera device 102, the lighting device 103, a computer system 20, an input-output interface device 30, a driving circuit 40, an output device 50, and an input device 60.

The computer system 20, the driving circuit 40, the output device 50, and the input device 60 perform data communication via the input-output interface device 30. The computer system 20 includes an arithmetic processing device 20A and a storage device 20B. The arithmetic processing device 20A includes a microprocessor, such as a central processing unit (CPU). The storage device 20B includes a memory or a storage, such as a read only memory (ROM) and a random access memory (RAM). The arithmetic processing device 20A performs an arithmetic process in accordance with a computer program 20C that is stored in the storage device 20B.

The driving circuit 40 generates a driving signal and outputs the driving signal to the display device 101, the stereo camera device 102, and the lighting device 103. Further, the driving circuit 40 supplies the image data of the eyeball 111 captured by the stereo camera device 102 to the computer system 20 via the input-output interface device 30.

The output device 50 includes a display device, such as a flat panel display. Meanwhile, the output device 50 may include a printing device. The input device 60 generates input data by being operated. The input device 60 includes a keyboard or a mouse for a computer system. Meanwhile, the input device 60 may include a touch sensor that is arranged on a display unit of the output device 50 as a display device.

In the present embodiment, the display device 101 and the computer system 20 are separated devices. Meanwhile, the display device 101 and the computer system 20 may be integrated with each other. For example, if the line-of-sight detection apparatus 100 includes a tablet personal computer, the computer system 20, the input-output interface device 30, the driving circuit 40, and the display device 101 may be mounted on the tablet personal computer.

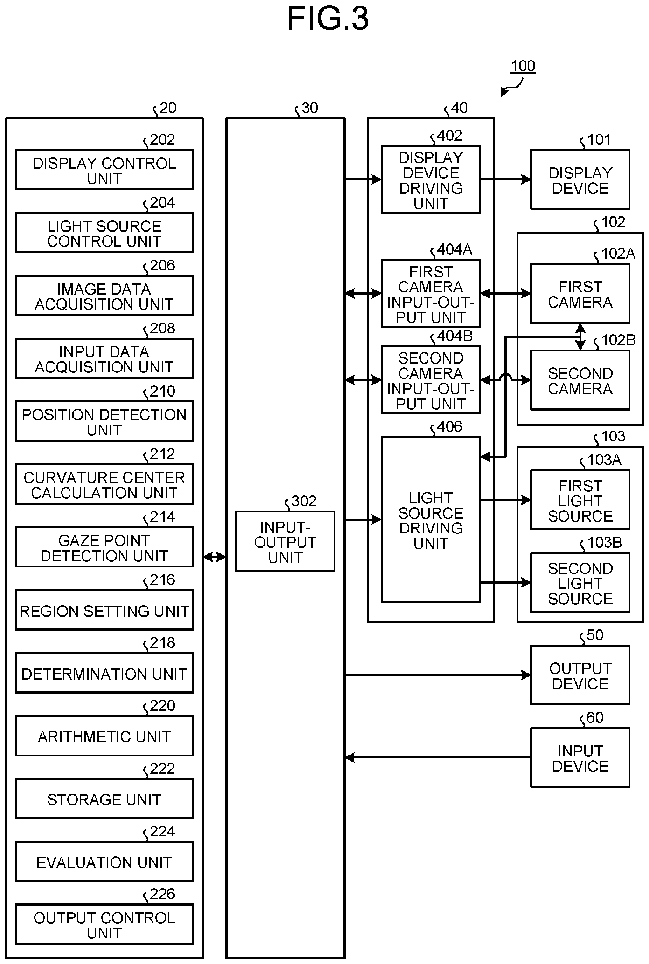

FIG. 3 is a functional block diagram illustrating an example of the line-of-sight detection apparatus 100 according to the present embodiment. As illustrated in FIG. 3, the input-output interface device 30 includes an input-output unit 302.

The driving circuit 40 includes a display device driving unit 402 that generates a driving signal for driving the display device 101 and outputs the driving signal to the display device 101, a first camera input-output unit 404A that generates a driving signal for driving the first camera 102A and outputs the driving signal to the first camera 102A, a second camera input-output unit 404B that generates a driving signal for driving the second camera 102B and outputs the driving signal to the second camera 102B, and a light source drive unit 406 that generates driving signals for driving the first light source 103A and the second light source 103B and outputs the driving signals to the first light source 103A and the second light source 103B. Further, the first camera input-output unit 404A supplies the image data of the eyeball 111 captured by the first camera 102A to the computer system 20 via the input-output unit 302. The second camera input-output unit 404B supplies the image data of the eyeball 111 captured by the second camera 102B to the computer system 20 via the input-output unit 302.

The computer system 20 controls the line-of-sight detection apparatus 100. The computer system 20 includes a display control unit 202, a light source control unit 204, an image data acquisition unit 206, an input data acquisition unit 208, a position detection unit 210, a curvature center calculation unit 212, a gaze point detection unit 214, a region setting unit 216, a determination unit 218, an arithmetic unit 220, a storage unit 222, an evaluation unit 224, and an output control unit 226. Functions of the computer system 20 are implemented by the arithmetic processing device 20A and the storage device 20B.

The display control unit 202 controls the display device driving unit 402 and causes the display unit 101S of the display device 101 to display an evaluation image to be viewed by the subject. The evaluation image includes a still image and a moving image. For example, a plurality of evaluation images are prepared. The display control unit 202 sequentially displays the plurality of evaluation images on the display device 101. Further, the display control unit 202 may cause the display device 101 to display an eye-catching video for causing a gaze point P to be located at a desired position on the display unit 101S.

The light source control unit 204 controls the light source drive unit 406 and controls operating states of the first light source 103A and the second light source 103B. The light source control unit 204 controls the first light source 103A and the second light source 103B such that the first light source 103A and the second light source 103B emit detection light at different timings.

The image data acquisition unit 206 acquires the image data of the eyeball 111 of the subject captured by the stereo camera device 102 including the first camera 102A and the second camera 102B, from the stereo camera device 102 via the input-output unit 302.

The input data acquisition unit 208 acquires input data that is generated by operation on the input device 60, from the input device 60 via the input-output unit 302.

The position detection unit 210 detects positional data of a pupil center on the basis of the image data of the eyeball 111 acquired by the image data acquisition unit 206. Further, the position detection unit 210 detects positional data of a corneal reflection center on the basis of the image data of the eyeball 111 acquired by the image data acquisition unit 206. The pupil center is a center of the pupil 112. The corneal reflection center is a center of the corneal reflection image 113. The position detection unit 210 detects the positional data of the pupil center and the positional data of the corneal reflection center for each of the left and right eyeballs 111 of the subject.

The curvature center calculation unit 212 calculates the positional data of the corneal curvature center of the eyeball 111 on the basis of the image data of the eyeball 111 acquired by the image data acquisition unit 206.

The gaze point detection unit 214 detects positional data of the gaze point P of the subject on the basis of the image data of the eyeball 111 acquired by the image data acquisition unit 206. In the present embodiment, the positional data of the gaze point P is positional data of an intersection point of a line-of-sight vector of the subject that is defined by the three-dimensional global coordinate system and the display unit 101S of the display device 101. The gaze point detection unit 214 detects a line-of-sight vector of each of the left and right eyeballs 111 of the subject on the basis of the positional data of the pupil center and the positional data of the corneal curvature center that are acquired from the image data of the eyeball 111. After detection of the line-of-sight vector, the gaze point detection unit 214 detects the positional data of the gaze point P indicating the intersection point of the line-of-sight vector and the display unit 101S.

The region setting unit 216 sets a determination region corresponding to the evaluation image that is displayed on the display unit 101S of the display device 101. The region setting unit 216 sets the determination region in a person, an object, a pattern, or the like included in the evaluation image. In the present embodiment, the region setting unit 216 sets the determination region in at least a part of the evaluation image on the display unit 101S. It is preferable to set a plurality of determination regions. If a natural image and a geometric image are displayed on the display unit 101S, the region setting unit 216 may set the determination regions in at least a part of a region in which the natural image is displayed and in at least a part of a region in which the geometric image is displayed, for example. It is preferable to set the determination regions with respect to objects that are included in an evaluation video. If the determination regions are set with respect to a natural image, it is preferable to set the determination region for each of objects, such as a person or a face of the person, for example. The determination regions are not displayed on the display unit 101S. Shapes of the determination regions may be, for example, rectangles, circles, ellipses, polygons, or the like, and are not specifically limited. Sizes of the plurality of determination regions may be different from one another. The plurality of determination regions may be set in an overlapping manner. It is preferable to set the determination regions in a certain region of the evaluation image, in which a gaze tendency is different between a subject who is likely to have ASD and a subject who is not likely to have ASD.

The determination unit 218 determines whether the gaze point P is present in the determination regions on the basis of the positional data of the gaze point P, and outputs determination data. For example, the determination unit 218 outputs the determination data in which a determination value of a determination region is set to "1" when determining that the gaze point P is present in the determination region, outputs the determination data in which the determination value is set to "0" when determining that the gaze point P is not present in the determination region. When determining that the gaze point P is present in the determination region, the determination unit 218 sets the determination value to "1" regardless of a gaze time or the number of times of gazing at the determination region. The determination unit 218 sets the determination value to "1" regardless of whether the gaze time is long or short. Even when determining that the gaze point P is present in the determination region multiple times because of movement back and forth between the determination region and other regions, the determination unit 218 sets the determination value to "1". The determination unit 218 determines whether the gaze point P is present in the determination region at predetermined time intervals, for example. The predetermined time interval may be, for example, a cycle (for example, every 20 milliseconds (msec)) of the frame synchronous signal output from the first camera 102A and the second camera 102B.

The arithmetic unit 220 calculates a total number of determination regions in which the gaze point P is present for a predetermined time, on the basis of the determination data of the determination unit 218. In other words, the arithmetic unit 220 calculates the number of determination regions in the evaluation image that are viewed by the subject for the predetermined time. The arithmetic unit 220 calculates the total number of the determination regions in which the gaze point P is present by cumulating the determination values of the respective determination regions.

The arithmetic unit 220 includes a management timer that manages a replay time of a video and a detection timer that detects an elapsed time since display of the video on the display unit 101S. The arithmetic unit 220 calculates gaze information corresponding to the positional data of the gaze point, on the basis of a determination result of the determination unit 218. The gaze information indicates, for example, information that visually represents the gaze point of the subject in the evaluation image. Specifically, the gaze information is a mark, such as a point, that represents the position of the gaze point in the evaluation image, a heat map, a bar chart representing a gaze rate, or the like. The heat map indicates, for example, information on a density of the gaze point of the subject in the evaluation image. The gaze rate indicates, for example, information that represents a rate of the gaze point in each of predetermined ranges that are divided ranges in the evaluation image.

The evaluation unit 224 calculates evaluation data of the subject on the basis of the total number of the determination regions in which the gaze point P is present, which is calculated by the arithmetic unit 220. The evaluation data is data for evaluating the number of times that the subject gazes at the determination regions displayed on the display unit 101S in display operation. The evaluation data evaluates, for example, the number of the determination regions that are gazed at, independent of a time and the number of times of gazing at the determination regions. The evaluation unit 224 may obtain the evaluation data by adding weight to each of the determination regions in which the gaze point P is present. The evaluation unit 224 further determines whether the evaluation data is equal to or larger than a predetermined threshold, and determines evaluation.

The storage unit 222 stores therein the determination data and the evaluation data as described above. Further, the storage unit 222 stores therein an evaluation program for causing a computer to execute a process of displaying an image, a process of detecting the position of the gaze point P of the subject who observes the display unit, a process of setting the determination regions in the display unit, a process of determining whether the gaze point P is present in each of the determination regions on the basis of the positional data of the gaze point P and outputting determination data, a process of obtaining evaluation data of the subject, and a process of outputting the evaluation data.

The output control unit 226 outputs data to at least one of the display device 101 and the output device 50.

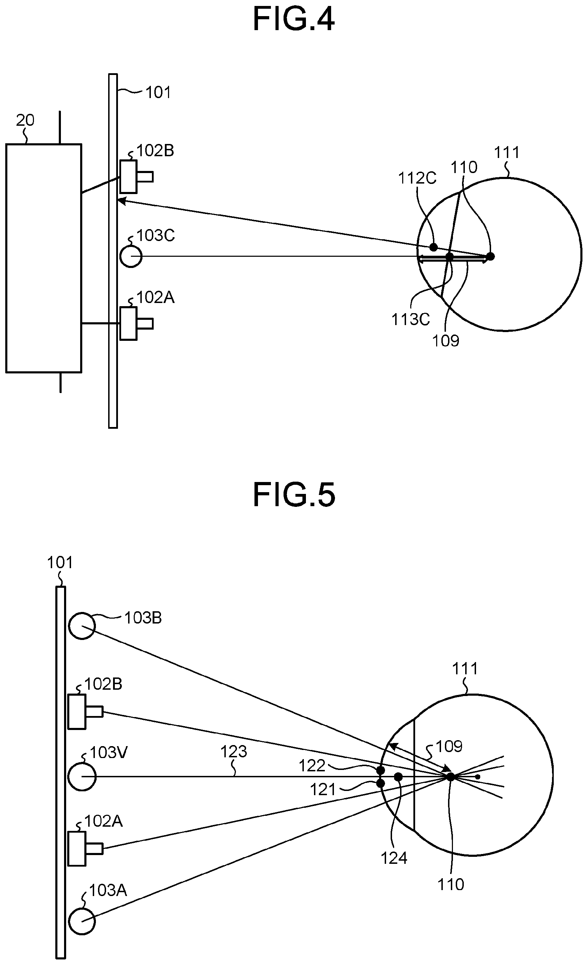

Next, an overview of a process performed by the curvature center calculation unit 212 according to the present embodiment will be described. The curvature center calculation unit 212 calculates the positional data of the corneal curvature center of the eyeball 111 on the basis of the image data of the eyeball 111. FIG. 4 and FIG. 5 are schematic diagrams for explaining a method of calculating positional data of a corneal curvature center 110 according to the present embodiment. FIG. 4 illustrates an example in which the eyeball 111 is illuminated by a single light source 103C. FIG. 5 illustrates an example in which the eyeball 111 is illuminated by the first light source 103A and the second light source 103B.

First, the example illustrated in FIG. 4 will be described. The light source 103C is arranged between the first camera 102A and the second camera 102B. A pupil center 112C is the center of the pupil 112. A corneal reflection center 113C is the center of the corneal reflection image 113. In FIG. 4, the pupil center 112C represents a pupil center in a case where the eyeball 111 is illuminated by the single light source 103C. The corneal reflection center 113C represents a corneal reflection center in the case where the eyeball 111 is illuminated by the single light source 103C. The corneal reflection center 113C is present on a straight line that connects the light source 103C and the corneal curvature center 110. The corneal reflection center 113C is located at an intermediate point between the cornea surface and the corneal curvature center 110. A corneal curvature radius 109 is a distance between the cornea surface and the corneal curvature center 110. Positional data of the corneal reflection center 113C is detected by the stereo camera device 102. The corneal curvature center 110 is present on a straight line that connects the light source 103C and the corneal reflection center 113C. The curvature center calculation unit 212 calculates, as the positional data of the corneal curvature center 110, positional data by which the distance from the corneal reflection center 113C on the straight line is equal to a predetermined value. The predetermined value is a value that is determined in advance from a curvature radius value of a general cornea, and stored in the storage unit 222.

Next, the example illustrated in FIG. 5 will be described. In the present embodiment, the first camera 102A and the second light source 103B are symmetrically arranged and the second camera 102B and the first light source 103A are symmetrically arranged with respect to a straight light that passes through an intermediate position between the first camera 102A and the second camera 102B. It is assumed that a virtual light source 103V is present at the intermediate position between the first camera 102A and the second camera 102B. A corneal reflection center 121 represents a corneal reflection center in an image of the eyeball 111 captured by the second camera 102B. A corneal reflection center 122 represents a corneal reflection center in an image of the eyeball 111 captured by the first camera 102A. A corneal reflection center 124 represents a corneal reflection center corresponding to the virtual light source 103V. Positional data of the corneal reflection center 124 is calculated on the basis of positional data of the corneal reflection center 121 and positional data of the corneal reflection center 122 that are captured by the stereo camera device 102. The stereo camera device 102 detects the positional data of the corneal reflection center 121 and the positional data of the corneal reflection center 122 in a three-dimensional local coordinate system defined in the stereo camera device 102. Camera calibration based on a stereo calibration method is performed in advance on the stereo camera device 102, and a conversion parameter for converting the three-dimensional local coordinate system of the stereo camera device 102 to a three-dimensional global coordinate system is calculated. The conversion parameter is stored in the storage unit 222. The curvature center calculation unit 212 converts the positional data of the corneal reflection center 121 and the positional data of the corneal reflection center 122 that are captured by the stereo camera device 102 to pieces of positional data in the three-dimensional global coordinate system by using the conversion parameter. The curvature center calculation unit 212 calculates positional data of the corneal reflection center 124 in the three-dimensional global coordinate system on the basis of the positional data of the corneal reflection center 121 and the positional data of the corneal reflection center 122 that are defined in the three-dimensional global coordinate system. The corneal curvature center 110 is present on a straight line 123 that connects the virtual light source 103V and the corneal reflection center 124. The curvature center calculation unit 212 calculates, as the positional data of the corneal curvature center 110, positional data by which a distance from the corneal reflection center 124 on the straight line 123 is equal to a predetermined value. The predetermined value is a value that is determined in advance from a curvature radius value of a general cornea, and stored in the storage unit 222.

As described above, even if the two light sources are provided, the corneal curvature center 110 is calculated by the same method as the method that is adopted when the single light source is provided.

The corneal curvature radius 109 is a distance between the cornea surface and the corneal curvature center 110. Therefore, the corneal curvature radius 109 is calculated by calculating the positional data of the cornea surface and the positional data of the corneal curvature center 110.

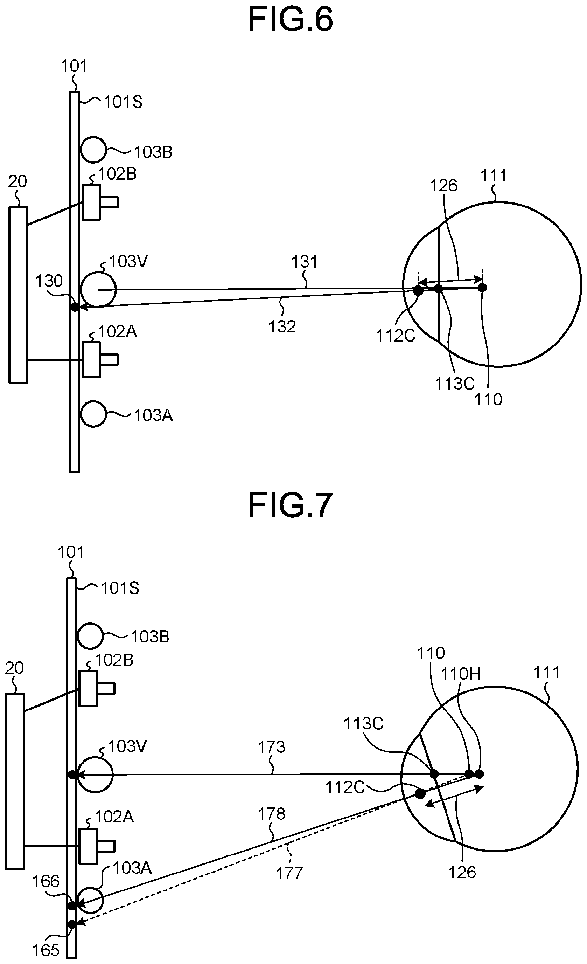

Next, an example of a line-of-sight detection method according to the present embodiment will be described. FIG. 6 is a schematic diagram for explaining an example of a calibration process according to the present embodiment. In the calibration process, a target position 130 is set so as to be gazed at by the subject. The target position 130 is defined in the three-dimensional global coordinate system. In the present embodiment, the target position 130 is set to a central position of the display unit 101S of the display device 101, for example. Meanwhile, the target position 130 may be set to an edge position of the display unit 101S. The output control unit 226 displays a target image at the set target position 130. A straight line 131 is a straight line that connects the virtual light source 103V and the corneal reflection center 113C. A straight line 132 is a straight line that connects the target position 130 and the pupil center 112C. The corneal curvature center 110 is an intersection point between the straight line 131 and the straight line 132. The curvature center calculation unit 212 is able to calculate the positional data of the corneal curvature center 110 on the basis of positional data of the virtual light source 103V, positional data of the target position 130, positional data of the pupil center 112C, and the positional data of the corneal reflection center 113C.

Next, a gaze point detection process performed by the gaze point detection unit 214 will be described. The gaze point detection process is performed after the calibration process. The gaze point detection unit 214 calculates a line-of-sight vector and the positional data of the gaze point P of the subject on the basis of the image data of the eyeball 111. FIG. 7 is a schematic diagram for explaining an example of the gaze point detection process according to the present embodiment. In FIG. 7, a gaze point 165 represents the gaze point P obtained from a corneal curvature center that is calculated using a general curvature radius value. A gaze point 166 represents the gaze point P obtained from a corneal curvature center that is calculated using a distance 126 obtained through the calibration process. The pupil center 112C represents a pupil center calculated in the calibration process, and the corneal reflection center 113C represents a corneal reflection center calculated through the calibration process. A straight line 173 is a straight line that connects the virtual light source 103V and the corneal reflection center 113C. The corneal curvature center 110 is a position of corneal curvature center that is calculated from a general curvature radius value. The distance 126 is a distance between the pupil center 112C and the corneal curvature center 110 calculated through the calibration process. A corneal curvature center 110H indicates a position of a corrected corneal curvature center obtained after the corneal curvature center 110 is corrected using the distance 126. The corneal curvature center 110H is obtained under a condition that the corneal curvature center 110 is present on the straight line 173 and the distance between the pupil center 112C and the corneal curvature center 110 is the distance 126. Accordingly, a line of sight 177 that is calculated when a general curvature radius value is used is corrected to a line of sight 178. Further, the gaze point P on the display unit 101S of the display device 101 is corrected from the gaze point 165 to a gaze point 166.

### Display method

Next, a display method according to the present embodiment will be described. In the display method according to the present embodiment, movement of the gaze point of the subject with respect to a video is evaluated by using the line-of-sight detection apparatus 100 as described above.

Recently, videos for supporting diagnosis of developmental disabilities have been actively developed. Here, it is needed to examine sensitivity and specificity of a video. It is needed to develop videos such that a person with typical development and a person with a developmental disability have different gaze patterns with respect to each of the videos. To cope with this, an apparatus for evaluating the above has been demanded.

A conventional gaze point measurement apparatus displays a video and examines the way that a subject views the video, but does not have a function to superimposing gaze points of a large number of subjects and examine tendencies. As a result, although it is possible to examine a portion that has been viewed by aggregating coordinates of gaze points, but it is difficult to dynamically obtain a tendency of how a gaze point moves in association with movement or the like of a video. In addition, in particular, it is difficult to examine what kind of difference occurs due to a gender difference, an age difference, or the like. To cope with this, the present disclosure is conceived to selectively display gaze points of a plurality of subjects on a replay screen of a stimulus video viewed by the subjects, and allow to dynamically check movement of the gaze points.

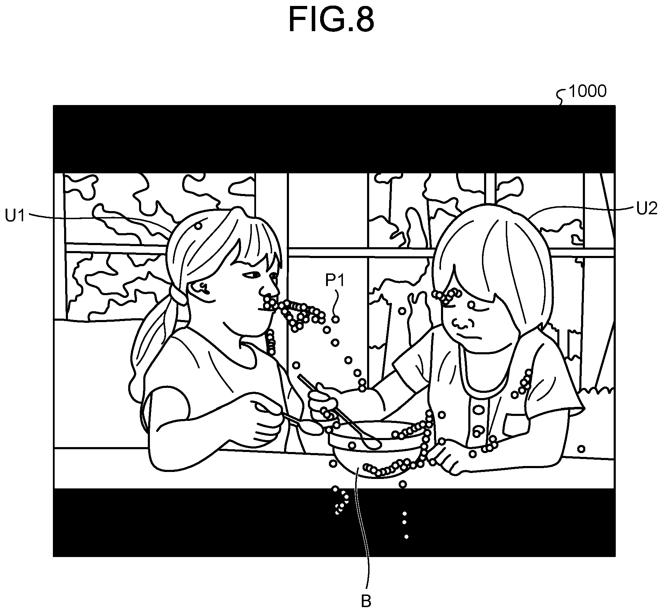

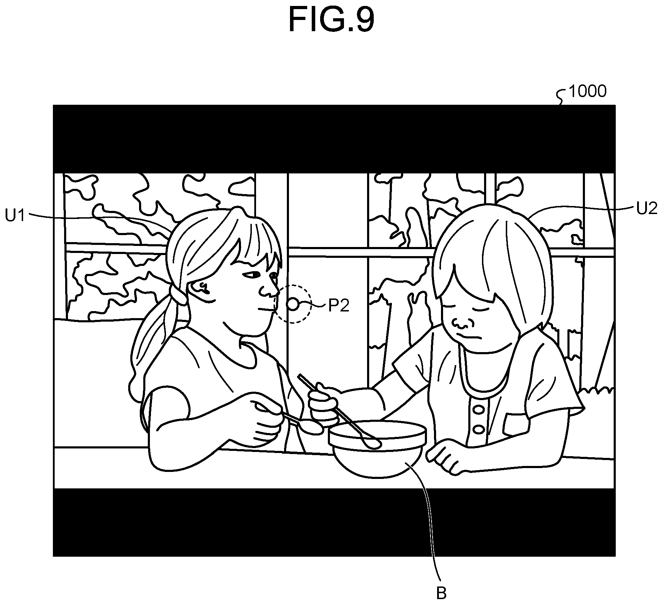

First, an example of conventional gaze point display with respect to a display image will be described with reference to FIG. 8 and FIG. 9. FIG. 8 is a schematic diagram for explaining an example of conventional gaze point display. FIG. 9 is a schematic diagram for explaining an example of a conventional screen for dynamically displaying gaze points. A video 1000 illustrated in FIG. 8 and FIG. 9 is a video including a person U1 and a person U2.

FIG. 8 illustrates a state in which gaze points P1 of a single subject from start to end of replay of the video are collectively displayed. In FIG. 8, it is indicated that the gaze points P1 are gathered in the vicinity of a face of the person U1, a bowl B, and the like. However, in the conventional example as described above, if gaze points of a plurality of subjects are displayed simultaneously, the gaze points are not easily viewable because they overlap with one another, and it is difficult to check temporal movement of the gaze points.

FIG. 9 illustrates one conventional display example in which a gaze point is dynamically displayed on a display screen. In the conventional example as described above, how a gaze point P2 moves is observed while replaying the video, on the basis of coordinates of the gaze point of the subject on the video. In the conventional example as described above, it is difficult to simultaneously display movement of gaze points of a plurality of subjects.

Next, the present disclosure will be described. In the present disclosure, it is possible to display pieces of gaze information on gaze points of a plurality of subjects, together with a video.

In the present embodiment, the gaze information is information that visually represents gaze movement of the subject on a video. For example, the gaze information in the present embodiment includes gaze points that are displayed together with the video, a heat map that is displayed together with the video, a bar chart that is displayed for each of grids that are divided grids in the video, or the like. In the following, a method of displaying, as the gaze information, the gaze points, the heat map, and the bar chart together with the video will be described. Meanwhile, the gaze points, the heat map, and the bar chart are mere examples of the gaze information, and the present disclosure is not limited thereto.

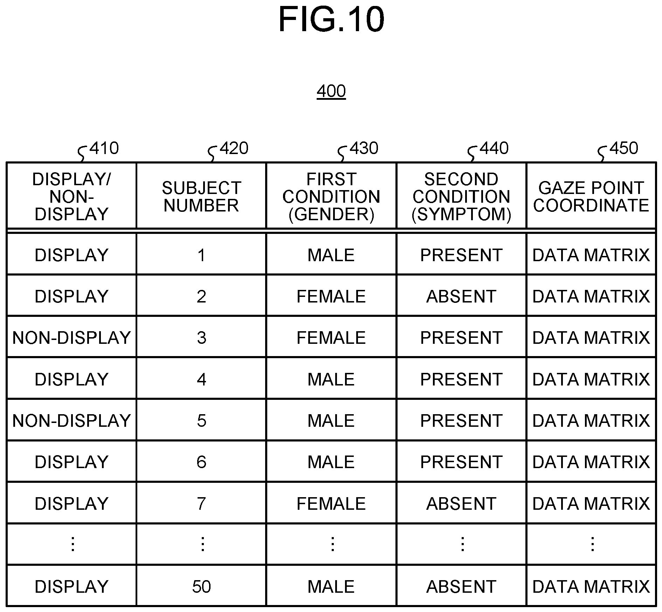

With reference to FIG. 10, a data set related to the gaze data of the subject according to the present embodiment will be described. FIG. 10 is a schematic diagram illustrating an example of the data set related to the gaze data of the subject according to the present embodiment.

As illustrated in FIG. 10, a data set 400 contains, for example, a display/non-display field 410, a subject number field 420, a first condition field 430, a second condition field 440, and a gaze point coordinate field 450. In the present embodiment, the data set 400 is stored in, for example, the storage unit 222.

In the display/non-display field 410, information indicating whether to display gaze points on a video is stored. If the display/non-display field 410 indicates display, the gaze points of the subject are displayed on the video. If the display/non-display field 410 indicates non-display, the gaze points of the subject are not displayed on the video.

In the subject number field 420, a number for identifying a subject is stored. In the data set 400, integers from 1 to 50 are stored in the subject number field 420. Meanwhile, while the integers from 1 to 50 are stored in the data set 400, this is a mere example, and the present disclosure is not limited thereto. In the present disclosure, the numbers in the subject number field are not specifically limited.

In the first condition field 430, information on a first condition is stored for each of subjects. The first condition is not specifically limited, but the first condition in the data set 400 is gender. For example, it is indicated that a subject with a subject number of 1 is a male, and a subject with a subject number of 2 is a female.

In the second condition field 440, information on a second condition is stored for each of the subjects. The second condition is not specifically limited, but the second condition in the data set 400 is presence or absence of a symptom, such as a developmental disability. For example, it is indicated that the subject with the subject number of 1 has a symptom, and the subject with the subject number of 2 does not have a symptom.

In the gaze point coordinate field 450, information on coordinates of gaze points of each of the subject with respect to the video is stored. In the present embodiment, the coordinates of the gaze points are stored as data matrix including coordinates on the display screen and time information.

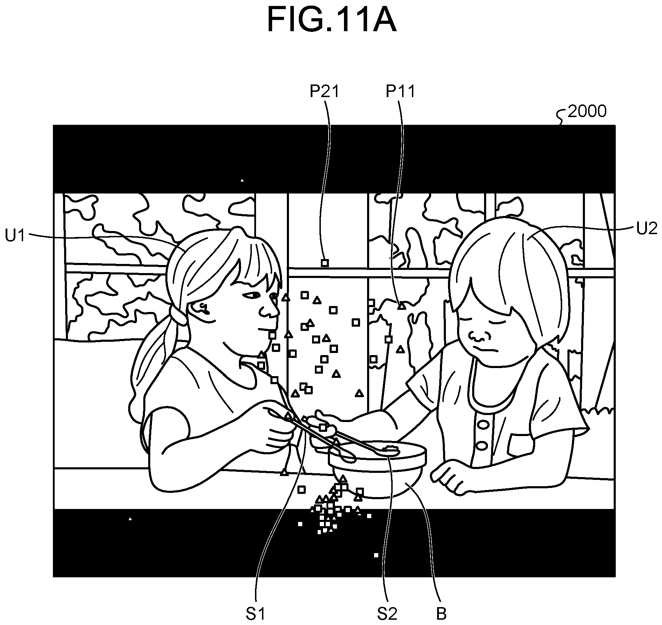

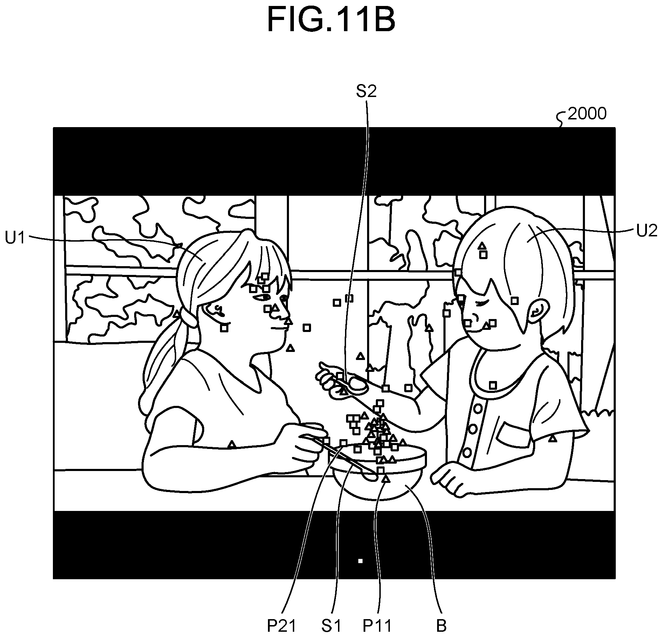

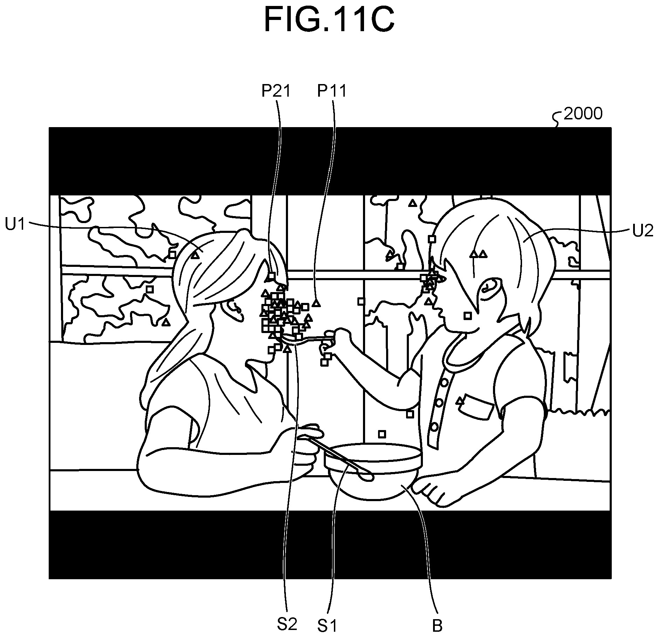

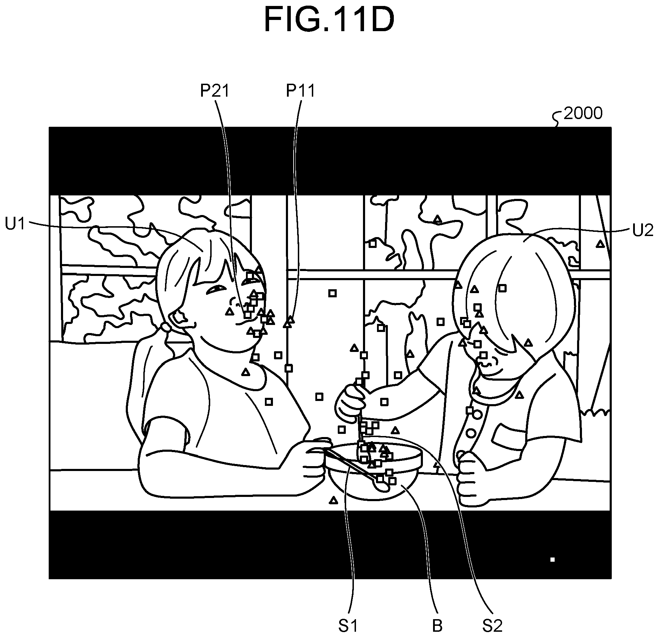

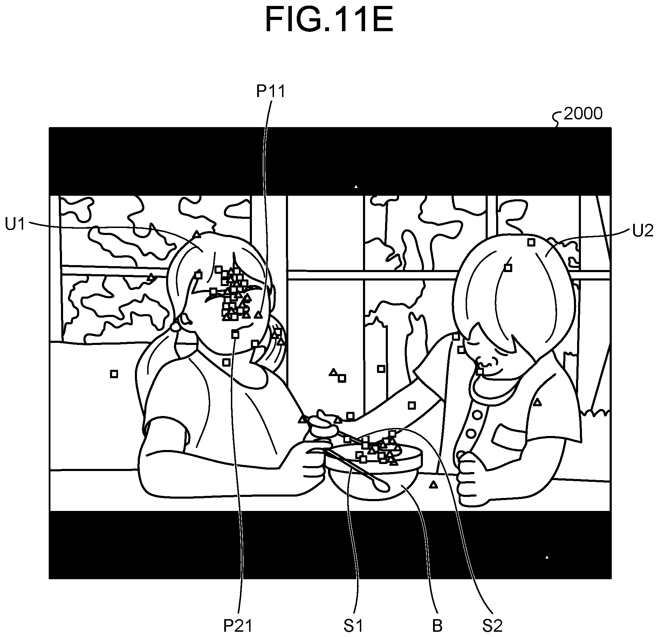

Examples of the method of displaying gaze points according to the present embodiment will be described with reference to FIG. 11A, FIG. 11B, FIG. 11C, FIG. 11D, and FIG. 11E. FIG. 11A to FIG. 11E are schematic diagrams for explaining the method of displaying gaze points on the display screen of the present embodiment.

FIG. 11A to FIG. 11E indicate a temporal change of a display video. Specifically, time goes from FIG. 11A to FIG. 11E in this order.

As illustrated in FIG. 11A, a video 2000 includes the person U1 and the person U2. The display control unit 202 displays information on gaze points of the plurality of subjects with respect to the video 2000, in a superimposed manner on the video 2000. In the video 2000, gaze points P11 represented by triangular marks indicate gaze points of a male subject. Gaze points P21 represented by rectangular marks indicate gaze points of a female subject. In other words, the display control unit 202 simultaneously displays the gaze points of the plurality of subjects under two different conditions such as a male and a female. In the video 2000, it is indicated that the gaze points P11 and the gaze points P21 are scattered between a face of the person U1 and a face of the person U2, and concentrated in the vicinity of a central portion of a black belt region that is located in the vicinity of and below the bowl B.

FIG. 11B indicates that the gaze points P11 and the gaze points P21 are concentrated at a portion in which the person U2 scoops a food in the bowl B with a spoon S2. In other words, FIG. 11B indicates that the gaze points P11 and the gaze points P21 are moved from FIG. 11A.

FIG. 11C illustrates that when the person U2 gives the food that has been scooped with the spoon S2 from the bowl B to the person U1, the gaze points P11 and the gaze points P21 are concentrated at a mouth of the person U1. In other words, FIG. 11C indicates that the gaze points P11 and the gaze points P21 are further moved from FIG. 11B.

FIG. 11D illustrates that when the person U2 is trying to scoop the food in the bowl B with the spoon S2 after the person U2 gives the food to the person U1, the gaze points P11 and the gaze points P21 are concentrated at the face of the person U1 and the bowl B. In other words, FIG. 11D indicates that the gaze points P11 and the gaze points P21 are further moved from FIG. 11C.

FIG. 11E illustrates that when the person U1 smiles after the person U2 gives the food to the person U1, the gaze points P11 and the gaze points P21 are concentrated at the face of the person U1. In other words, FIG. 11E indicates that the gaze points P11 and the gaze points P21 are further moved from FIG. 11D.

As illustrated in FIG. 11A to FIG. 11E, the display control unit 202 displays the gaze points P11 and the gaze points P21 in a superimposed manner on the video 2000 in accordance with the elapsed time of the video 2000. Specifically, the display control unit 202 displays movement of the coordinate positions of the gaze points P11 and the gaze points P21 along with the elapsed time of the video 2000. Coordinate data of the coordinate positions of the gaze points P11 and the gaze points P21 are calculated by, for example, the arithmetic unit 220.

In FIG. 11A to FIG. 11E, the gaze points of the male and the female are displayed in a superimposed manner on the video, but this is a mere example, and the present disclosure is not limited thereto. In the present disclosure, it may be possible to superimpose, as the gaze information, a heat map on the video, instead of the gaze points.

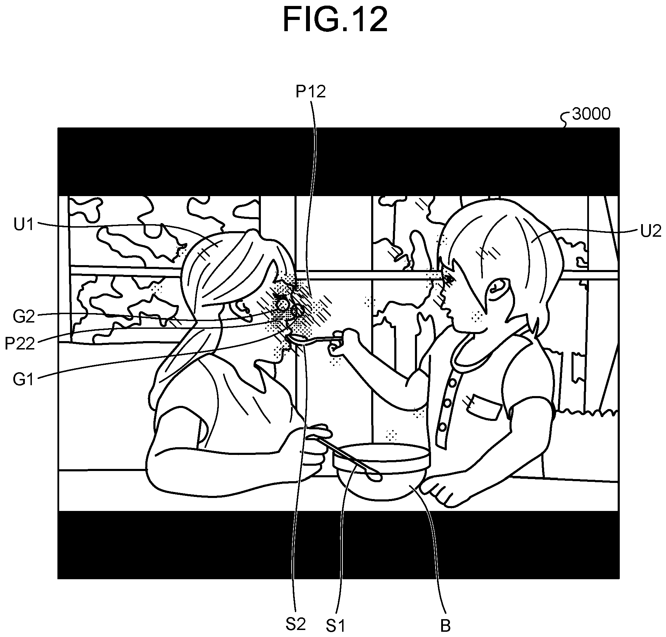

An example of the method of displaying the gaze points according to the present embodiment will be described with reference to FIG. 12. FIG. 12 is a schematic diagram for explaining an example of a method of displaying gaze points on the display screen of the present embodiment, which is different from the method as illustrated in FIG. 11A to FIG. 11E.

As illustrated in FIG. 12, the display control unit 202 may display a heat map of gaze points in a superimposed manner on a video 3000 in accordance with an elapsed time of the video. In this case, the arithmetic unit 220 calculates the heat map with respect to the video 3000 on the basis of gaze point data of each of the subjects stored in the storage unit 222.

The display control unit 202 displays, as heat maps, a gaze region P12 and a gaze region P22 in a superimposed manner on the video 3000 on the basis of an arithmetic result of the arithmetic unit 220. Here, the gaze region P12 represented by oblique lines is a gaze region of a male subject, and the gaze region P22 represented by dots is a gaze region of a female subject. The display control unit 202 displays a first centroid G1 that is a centroid of gaze points of the male and a second centroid G2 that is a centroid of gaze points of the female in a superimposed manner on the video 3000. When displaying the gaze regions by oblique lines and dots, the display control unit 202 displays the gaze regions with different densities depending on a degree of overlap between the gaze regions, for example. In this case, the display control unit 202 increases the densities with an increase in the degree of overlap when performing display. The display control unit 202 may display the gaze region P12 and the gaze region P22 in different colors. In this case, the display control unit 202 displays the gaze region P12 and the gaze region P22 with different saturation depending on the degree of overlap between the gaze regions, for example. The display control unit 202 increases saturation with an increase in the degree of overlap between the gaze regions when performing display, for example. With this configuration, it is possible to easily check the regions gazed at by the subjects in the video 3000. For example, by superimposing the gaze region P12 and the gaze region P22 in a semi-transparent manner on the video 3000, the display control unit 202 makes it possible to easily recognize how the gaze region P12 and the gaze region P22 overlap with each other.

In the present disclosure, as display of gaze points with respect to a video, for example, it may be possible to divide the entire display screen into lattices, and display a gaze rate for each of the lattices by a bar chart or the like. For example, in the present disclosure, each of the lattices is drawn by grid lines on the display screen. Therefore, in the following, the lattices on the display screen may be referred to as grids.

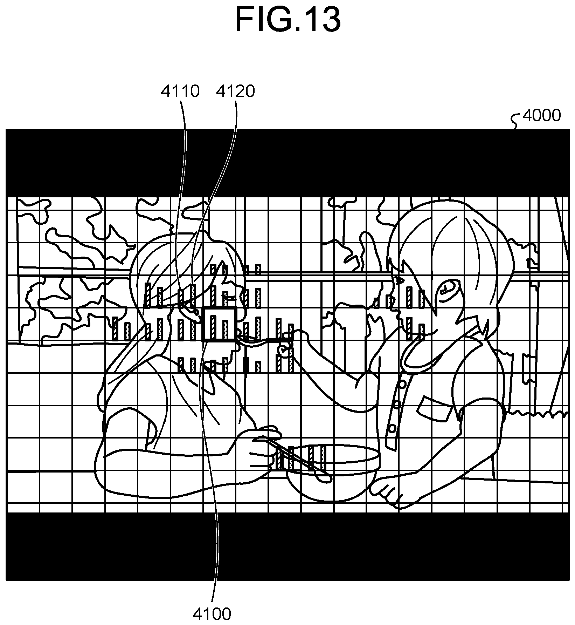

An example of the method of displaying gaze points according to the present embodiment will be described with reference to FIG. 13. FIG. 13 is a schematic diagram for explaining an example of a method of displaying gaze points on the display screen of the present embodiment, which is different from the methods as illustrated in FIG. 11A to FIG. 11E and FIG. 12.

As illustrated in FIG. 13, the display control unit 202 may divide a video 4000 into a plurality of grids 4100 on the display screen, and display a bar chart 4110 and a bar chart 4120 in each of the grids 4100. The bar chart 4110 represents a gaze rate of a male with respect to a corresponding grid, and the bar chart 4120 represents a gaze rate of a female with respect to the corresponding grid. The bar chart 4110 and the bar chart 4120 may be displayed in different colors, or may be displayed using different kinds of hatching. In this case, the arithmetic unit 220 calculates the gaze rate of each of the grids for each of the male and the female, on the basis of gaze point data for each of the subjects stored in a storage unit 228. Further, it is preferable that the arithmetic unit 220 normalizes the gaze rate by the number of males or females.

The display control unit 202 divides the video 4000 into the plurality of grids 4100 and displays the bar chart 4110 and the bar chart 4120 in each of the grids 4100, on the basis of the arithmetic result of the arithmetic unit 220. The display control unit 202 calculates the gaze rates for each replay time of the video 4000, and displays the bar chart 4110 and the bar chart 4120. In other words, the bar chart 4110 and the bar chart 4120 that are displayed in a superimposed manner on the video 4000 are changed depending on the replay time of the video 4000. The display control unit 202 displays the normalized bar chart 4110 and the normalized bar chart 4120 in each of the grids 4100, so that it becomes easy to compare the gaze rates.

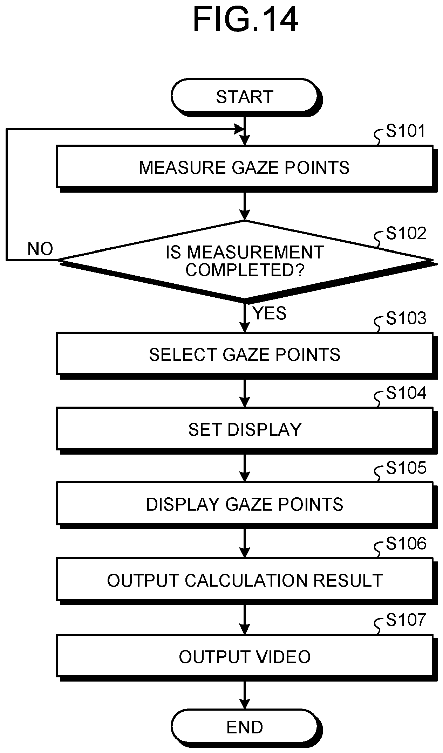

The method of displaying gaze points on a video will be described with reference to FIG. 14. FIG. 14 is a flowchart illustrating an example the flow of a process of displaying gaze points on a video.

First, the gaze point detection unit 214 measures gaze points of the subject with respect to the video (Step S101). Subsequently, at Step S102, the gaze point detection unit 214 determines whether gaze points of all of the subjects with respect to the video are measured. If the gaze points of all of the subjects with respect to the video are not measured (No at Step S102), the gaze point detection unit 214 returns to Step S101 and continues the process. In contrast, if the gaze points of all of the subjects with respect to the video are measured (Yes at Step S102), the gaze point detection unit 214 proceeds to Step S103.

Meanwhile, if the gaze points of all of the subjects are measured in advance, Step S101 and Step S102 may be omitted.

Subsequently, the display control unit 202 receives measurement data of gaze points of a subject that is selected and needed to be displayed among the calculated gaze points (Step S103).

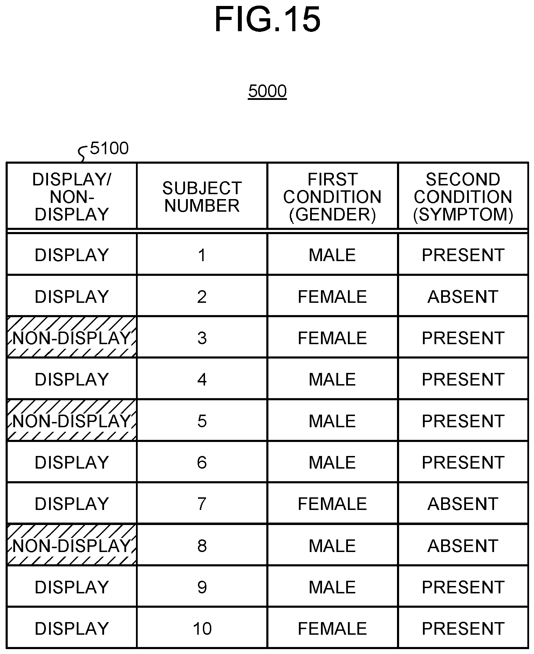

A method of selecting a subject for which the measurement data need to be displayed will be described with reference to FIG. 15. FIG. 15 is a schematic diagram for explaining an example of the method of selecting a subject to be displayed on the video.

FIG. 15 illustrates a selection screen 5000 for selecting display and non-display among the pieces of calculated measurement data of the subjects. The selection screen 5000 includes a display/non-display button 5100. In this case, for example, a user is able to set display and non-display for each of the subjects by operating an operation unit, such as a mouse, and clicking the display/non-display button 5100. In the selection screen 5000, non-display is set for subjects with subject numbers 3, 5, and 8. In the example illustrated in the selection screen 5000, when the display/non-display button 5100 indicates non-display, oblique lines are drawn so as to be easily distinguished from a case of display.

Referring back to FIG. 14, the display control unit 202 sets a type of gaze information to be displayed on the video (Step S104).

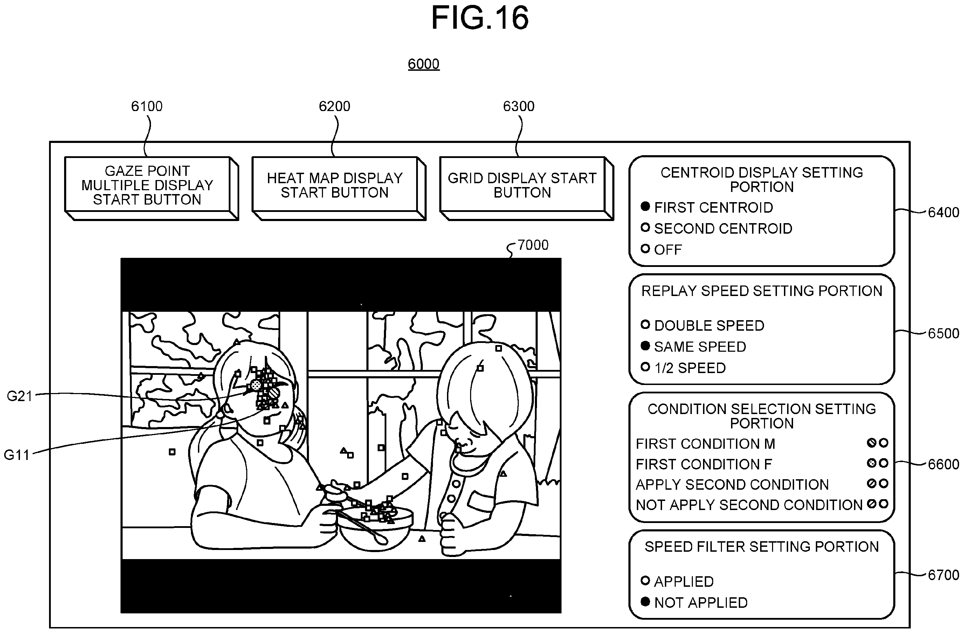

A method of setting the type of the gaze information to be displayed on the video will be described with reference to FIG. 16. FIG. 16 is a schematic diagram for explaining an example of the method of selecting the type of the gaze information to be displayed on the video.

An interface 6000 is one example of a setting screen for selecting the type of the gaze information to be displayed on the video. In a central portion of the interface 6000, a video in which a video 7000 and gaze points of a plurality of subjects are superimposed is displayed. In an upper portion of the interface 6000, three start buttons, in particular, a gaze point multiple display start button 6100, a heat map display start button 6200, and a grid display start button 6300 are arranged. In a right portion of the interface 6000, a centroid display setting portion 6400, a replay speed setting portion 6500, a condition selection setting portion 6600, and a speed filter setting portion 6700 are provided.

In the present embodiment, the user is able to select a desired display mode on the interface 6000. If the gaze point multiple display start button 6100 is selected, the video 2000 as illustrated in FIG. 11A to FIG. 11E is displayed, for example. If the heat map display start button 6200 is selected, the video 3000 as illustrated in FIG. 12 is displayed, for example. If the grid display start button 6300 is selected, the video 4000 as illustrated in FIG. 13 is displayed, for example. In other words, the display control unit 202 selectively displays a plurality of pieces of gaze information in different display modes, in accordance with an instruction from the user.

In the centroid display setting portion 6400, is is possible to display a centroid of gaze points. The centroid of the gaze points will be described later.

In the replay speed setting portion 6500, it is possible to set a replay speed of the video 7000. As the replay speed, for example, it may be possible to set a double speed, the same speed, 1/2 speed, or the like. In the replay speed setting portion 6500, it is possible to set an appropriate replay speed when performing an analysis in accordance with the video to be replayed.

In the condition selection setting portion 6600, it is possible to set a condition of gaze points to be displayed on the video 7000. In the present embodiment, if a first condition M is selected, gaze points of a male subject are displayed. If a second condition F is selected, gaze points of a female subject are displayed. If application of a second condition is selected, gaze points of a subject with a symptom are displayed. If non-application of the second condition is selected, gaze points of a subject without a symptom are displayed.

In the video 7000, if the gaze points of the subjects are to be examined, or if the centroids of the gaze points are calculated, movement (saccade) of gaze points at a high speed may have an adverse effect. Therefore, in the speed filter setting portion 6700, it is possible to set a filter for cancelling gaze points that move at a certain speed equal to or higher than the predetermined speed.

Referring back to FIG. 14, the display control unit 202 displays the gaze points in a superimposed manner on the video in accordance with a display method selected at Step S104 (Step S105).

Then, the output control unit 226 outputs a calculation result of the gaze points as a data file (Step S106). Further, the output control unit 226 outputs, as a video file, a video in which the gaze points are displayed in a superimposed manner on the video (Step S107). Then, the process in FIG. 14 is terminated.

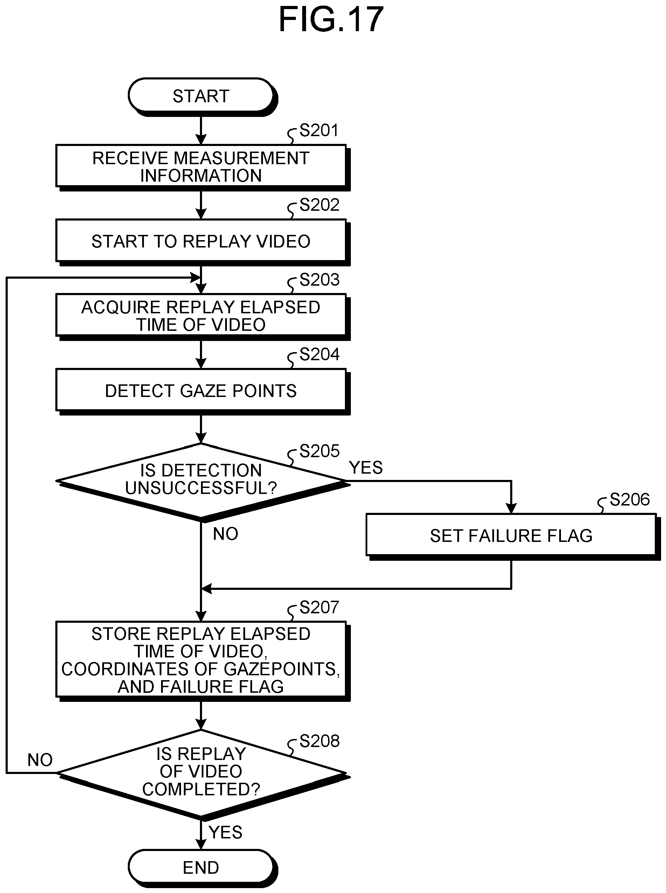

Next, a method of detecting gaze points on a video will be described with reference to FIG. 17. FIG. 17 is a flowchart illustrating an example of the flow of a process of detecting gaze points.

First, the arithmetic processing device 20A receives various kinds of measurement information on measurement of gaze points of a subject (Step S201). Specifically, the arithmetic processing device 20A receives various kinds of information included in the data set 400 illustrated in FIG. 10.

Subsequently, the arithmetic processing device 20A causes the display control unit 202 to start to replay a video (Step S202). The arithmetic processing device 20A causes the display control unit 202 to acquire an elapsed time since start of the replay of the video (Step S203).

The arithmetic processing device 20A causes the gaze point detection unit 214 to detect gaze points of the subject with respect to the video (Step S204). The arithmetic processing device 20A causes the gaze point detection unit 214 to determine whether detection of the gaze points is successful at Step S205.

If detection of the gaze points is unsuccessful (Yes at Step S205), the gaze point detection unit 214 proceeds to Step S206, and sets a failure flag (Step S206). Then, the gaze point detection unit 214 proceeds to Step S207. In contrast, if detection of the gaze points is successful (No at Step S205), the gaze point detection unit 214 proceeds to Step S207.

The arithmetic processing device 20A causes the gaze point detection unit 214 to store a replay elapsed time of the video and coordinates of the detected gaze points in the storage unit 222 (Step S207). If the failure flag is set, the gaze point detection unit 214 stores the failure flag in the storage unit 222.

Subsequently, at Step S208, the arithmetic processing device 20A causes the display control unit 202 to determine whether replay of the video is completed.

If replay of the video is not completed (No at Step S208), the display control unit 202 returns to Step S203 and repeats the above-described process. In contrast, if replay of the video is completed (Yes at Step S208), the display control unit 202 terminates the process in FIG. 17.

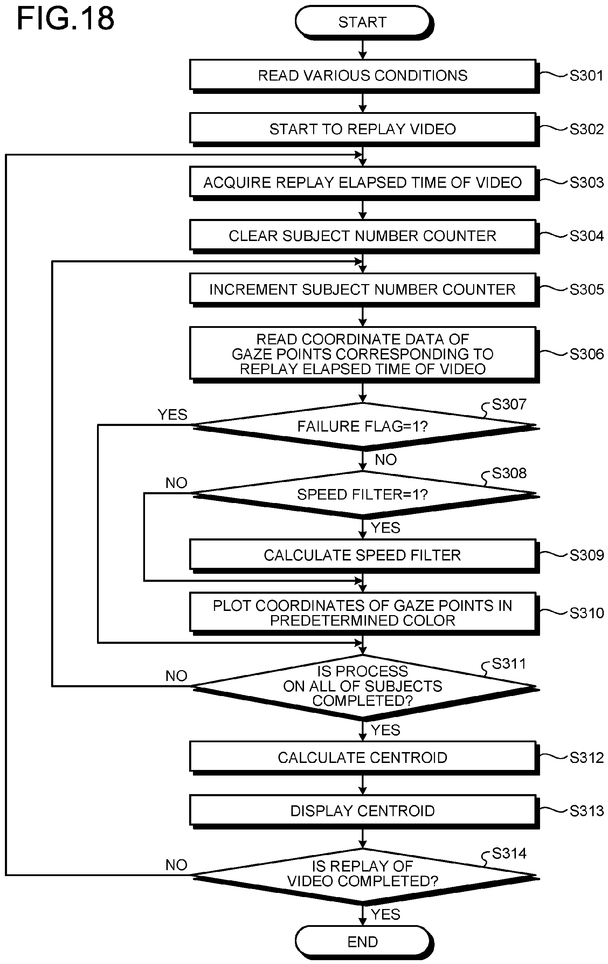

Next, a method of performing multiple display of gaze points on a video will be described with reference to FIG. 18. FIG. 18 is a flowchart illustrating an example of the flow of a process of performing multiple display of gaze points on a video.

First, the arithmetic processing device 20A reads various conditions for displaying gaze points (Step S301). Various conditions include various kinds of information included in the data set 400 as illustrated in FIG. 10 and information on the number of subjects.

Subsequently, the arithmetic processing device 20A causes the display control unit 202 to start to replay a video (Step S302). The display control unit 202 acquires an elapsed time since start of the replay of the video (Step S303).

Then, the arithmetic processing device 20A clears a subject number counter (Step S304). Specifically, the arithmetic processing device 20A clears the subject number counter such that the subject number counter H = 0. Here, the subject number counter H is a counter that is used to sequentially select and extract pieces of data on gaze points of a subject to be displayed.

Subsequently, the arithmetic processing device 20A increments the subject number counter (Step S305). Specifically, the arithmetic processing device 20A performs increment such that the subject counter H = H + 1.

Then, the arithmetic processing device 20A reads coordinate data of the gaze points corresponding to the replay elapsed time of the video from a data matrix corresponding to the subject number counter (Step S306). The arithmetic processing device 20A is able to read the coordinate data on the basis of the data set 400 as illustrated in FIG. 10. The coordinate data read by the arithmetic processing device 20A includes information on the failure flag.

Subsequently, at Step S307, the arithmetic processing device 20A determines whether the failure flag = 1 (failure). If the failure flag = 0 (success) (No at Step S307), the arithmetic processing device 20A proceeds to Step S308. In contrast, if the failure flag = 1 (Yes at Step S307), the arithmetic processing device 20A proceeds to Step S311.

Then, at Step S308, the arithmetic processing device 20A determines whether a speed filter is set. If the speed filter = 1 (already set) (Yes at Step S308), the arithmetic processing device 20A proceeds to Step S309. In contrast, if the speed filter = 0 (not set) (No at Step S308), the arithmetic processing device 20A proceeds to Step S310.

Subsequently, after Yes at Step S308, the arithmetic processing device 20A calculates the speed filter (Step S309). Specifically, the arithmetic processing device 20A calculates a distance from a position (coordinate) of the previously measured gaze point to a position (coordinate) of the currently measured gaze point among coordinates of gaze points that are measured at predetermined time intervals. Then, if the calculated distance is equal to or larger than a predetermined distance, the arithmetic processing device 20A determines that the data is not to be displayed.

Subsequently, the arithmetic processing device 20A plots and displays the coordinates of the gaze points corresponding to the replay elapsed time of the video in a predetermined color and in a superimposed manner on the video (Step S310).

Then, at Step S311, the arithmetic processing device 20A determines whether the process on all of the subjects is completed. Specifically, the arithmetic processing device 20A determines whether the subject number counter H indicates the number equal to or larger than the number of the subjects.

If the process on all of the subjects is completed (Yes at Step S311), the arithmetic processing device 20A proceeds to Step S312. In contrast, if the process on all of the subjects is not completed (No at Step S311), the arithmetic processing device 20A returns to Step S305 and repeats the process as described above.

Subsequently, the arithmetic processing device 20A calculates the centroid of the gaze points (Step S312). Specifically, the arithmetic processing device 20A calculates the centroid that is set in the centroid display setting portion 6400 as illustrated in FIG. 16. In FIG. 16, a first centroid G11 is the centroid of gaze points in a set of a predetermined number or more of gaze points located within a predetermined distance. At the first centroid G11, if gaze points are collectively scattered in a plurality of places, it is possible to obtain a centroid for each of scattered positions. Further, a second centroid G21 is a centroid of the entire gaze points for each of setting conditions (for example, male or female) used for display, independent of the scattered positions of the gaze points. At the second centroid G21, it is possible to obtain an average gaze position of a corresponding subject.

Subsequently, the arithmetic processing device 20A displays a plot at the position of the centroid calculated at Step S312, and displays the plot in a superimposed manner on the video (Step S313). Specifically, the arithmetic processing device 20A causes the display control unit 202 to display the centroid of a gaze point or the centroid of gaze points in a set of a predetermined number or more of gaze points, in a superimposed manner on the video.

Then, at Step S314, the arithmetic processing device 20A determines whether replay of the video is completed.

If replay of the video is not completed (No at Step S314), the arithmetic processing device 20A returns to Step S303, and repeats the process as described above. In contrast, if replay of the video is completed (Yes at Step S314), the arithmetic processing device 20A terminates the process in FIG. 18.

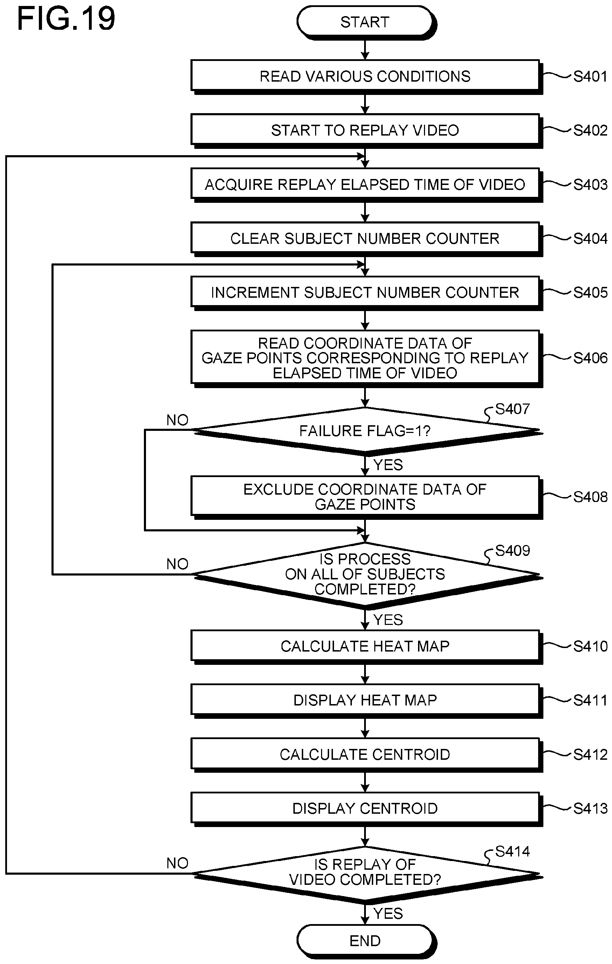

Next, a method of displaying a heat map on a video will be described with reference to FIG. 19. FIG. 19 is a flowchart illustrating an example of the flow of a process of displaying a heat map of gaze points on a video.

Processes from Step S401 to Step S407 are the same as the processes from Step S301 to Step S307 illustrated in FIG. 18, and therefore, explanation thereof will be omitted.

After No at Step S407, the arithmetic processing device 20A proceeds to Step S409. In contrast, after Yes at Step S407, the arithmetic processing device 20A proceeds to Step S408.

Subsequently, the arithmetic processing device 20A excludes the coordinate data of gaze points for which failures are determined (Step S408).

Then, at Step S409, the arithmetic processing device 20A determines whether a process on all of the subjects is completed.

If the process on all of the subjects is completed (Yes at Step S409), the arithmetic processing device 20A proceeds to Step S410. In contrast, if the process on all of the subjects is not completed (No at Step S409), the arithmetic processing device 20A returns to Step S405 and repeats the process as described above.

Subsequently, the arithmetic processing device 20A calculates the heat map of the gaze points in accordance with the set information (Step S410). Specifically, as illustrated in FIG. 12, the arithmetic processing device 20A changes the saturation of the heat map in accordance with the number of gaze points.

Then, the arithmetic processing device 20A displays the calculated heat map on the video in a superimposed manner (Step S411).

Step S412 to Step S414 are the same as Step S312 to Step S314 as illustrated in FIG. 18, and therefore, explanation thereof will be omitted.

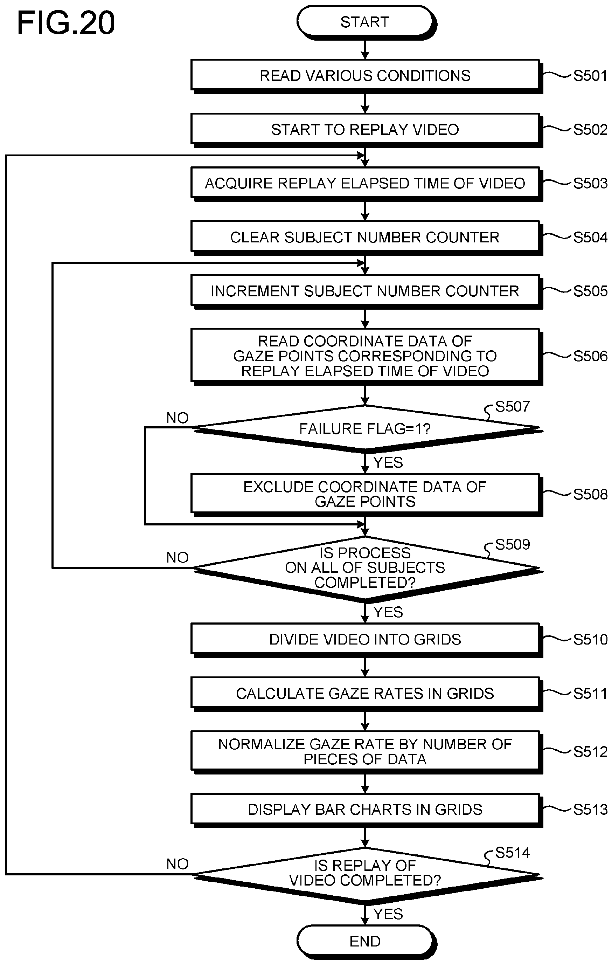

Next, a method of displaying gaze rates for each of grids on a video will be described with reference to FIG. 20. FIG. 20 is a flowchart illustrating an example of the flow of a process of displaying gaze rates for each of grids on a video.

Step S501 to Step S509 are the same as Step S401 to Step S409 as illustrated in FIG. 19.

After Yes at Step S509, the arithmetic processing device 20A divides the video into grids (Step S510). Here, a size of each of the grids and the number of the grids are arbitrary. Specifically, the arithmetic processing device 20A is able to divide into grids for each of regions in which gaze points are to be evaluated in the video.

Subsequently, the arithmetic processing device 20A calculates gaze points in each of the grids in accordance with set information (Step S511). Then, the arithmetic processing device 20A normalizes a gaze rate by the number of pieces of data of the gaze points (Step S512). For example, the line-of-sight detection apparatus 100 accumulates the gaze rates in the grid for each of a male and a female, and perform normalization by each of the number of males and the number of females. With this configuration, even if the number of males and the number of females are different, the arithmetic processing device 20A is able to compare the gaze rates of the male and the female.

Then, the arithmetic processing device 20A displays the calculated and normalized gaze rates by bar charts for each of the grids (Step S513). For example, as illustrated in FIG. 13, the arithmetic processing device 20A displays the gaze rate by the bar chart for each of the male and the female.

Subsequently, at Step S514, the arithmetic processing device 20A determines whether replay of the video is completed.

If replay of the video is not completed (No at Step S514), the arithmetic processing device 20A returns to Step S503, and repeats the process as described above. In contrast, if replay of the video is completed (Yes at Step S514), the arithmetic processing device 20A terminates the process in FIG. 20.

As described above, in the present disclosure, it is possible to display a gaze tendency of each of the subjects with respect to a video, in a superimposed manner on the video. Specifically, in the present disclosure, it is possible to display gaze points on the video, display a heat map, and display gaze rates for each of grids that are divided grids in the video. With this configuration, in the present disclosure, it is possible to examine gaze tendencies of subjects with respect to a video. Specifically, it is possible to easily and visually recognize how the gaze points are changed with a change of the video.

In particular, in the present disclosure, by displaying the gaze rates for each of the grids, it is possible to compare the gaze rates of subject groups that are classified by a specific condition. With this configuration, it is possible to more accurately examine a gaze tendency of a subject with respect to the video for each of the subjects.

With use of the present disclosure, by simultaneously displaying a gaze pattern of a person with a developmental disability and a gaze pattern of the subject in a distinguished manner, it is possible to evaluate a tendency of the developmental ability of the subject, and it is possible to support diagnosis of the developmental disability.

In the embodiments of the present disclosure, explanation is given using data of a developmental disability, but the present disclosure is not limited to the developmental disability, and may be adopted when evaluation on cognitive impairment, brain impairment or the like for which gaze points are used is to be performed.

While the embodiments of the present disclosure have been described above, the present disclosure is not limited to the contents of the embodiments as described above. Further, structural elements described above include one that can easily be thought of by a person skilled in the art, one that is practically identical, and one that is within an equivalent range. Furthermore, the structural elements as described above may be combined appropriately. Moreover, within the scope not departing from the gist of the embodiments as described above, various omission, replacement, and modifications of the structural elements may be made.

### Industrial Applicability

A display device, a display method, and a program according to the present disclosure may be used for an apparatus that displays distributions of lines of sight of subjects with respect to a video together with the video and examines gaze tendencies.

### Reference Signs List

- 20: Computer system

- 100: Line-of-sight detection apparatus
- 101: Display device
- 101S: Display unit
- 102: Stereo camera device
- 103: Lighting device
- 202: Display control unit
- 214: Gaze point detection unit
- 216: Region setting unit
- 218: Determination unit
- 220: Arithmetic unit
- 222: Storage unit
- 224: evaluation unit
- 226: Output control unit

## Claims

1. A display device comprising:
a storage unit configured to store positional data of a gaze point of a subject with respect to a video;
an arithmetic unit configured to calculate gaze information that visually indicates the gaze point from the positional data; and
a display control unit configured to cause a display screen of a display unit to display the gaze information in a superimposed manner on the video in accordance with a replay elapsed time of the video.

2. The display device according to claim 1, wherein
the arithmetic unit is configured to calculate a plurality of pieces of gaze information having different display modes, and
the display control unit is configured to display a piece of gaze information that is selected from the plurality of pieces of gaze information in accordance with a condition set by a user, in a superimposed manner on the video.

3. The display device according to claim 1 or 2, wherein the display control unit is configured to divide the display screen into grids, and display gaze rates of a plurality of subjects in predetermined time units for each of the grids.

4. The display device according to claim 1 or 2, wherein
the arithmetic unit is further configured to calculate one of a centroid of gaze points and a centroid of a set of a predetermined number or more of gaze points, at predetermined time intervals, and
the display control unit is configured to display one of the centroid of gaze points and the centroid of the set of the predetermined number or more of gaze points.

5. A display method comprising:
a calculation step of calculating gaze information that visually indicates a gaze point from positional data of the gaze point of a subject with respect to a video; and
a display control step of causing a display screen of a display unit to display the gaze information in a superimposed manner on the video in accordance with a replay elapsed time of the video.

6. A program that causes a computer to execute:
a calculation step of calculating gaze information that visually indicates a gaze point from positional data of the gaze point of a subject with respect to a video; and
a display control step of causing a display screen of a display unit to display the gaze information in a superimposed manner on the video in accordance with a replay elapsed time of the video.
